# EUROPEAN PATENT SPECIFICATION

(11) **EP 2 666 502 B1**
(45) Date of publication and mention of the grant of the patent: **29.06.2016**
(21) Application number: 13169259.2
(22) Date of filing: 24.05.2013
(51) Int. Cl.: A61M 15/00

(54) **An inhaler having a mechanism with double actuating levers**
Inhalator mit einem Mechanismus mit doppelter Hebelbetätigung
Inhalateur comportant un mécanisme à leviers d'actionnement doubles

(30) Priority: 25.05.2012 TR 201206167; 08.02.2013 TR 201301562; 15.02.2013 TR 201301847; 19.02.2013 TR 201301950; 26.03.2013 TR 201303661; 29.04.2013 TR 201305053
(43) Date of publication of application: 27.11.2013
(73) Proprietor: Arven Ilac Sanayi Ve Ticaret A.S., Istanbul 34460 (TR)
(72) Inventor: Toksöz, Zafer, 34460 Istanbul (TR); Cifter, Ümit, 34460 Istanbul (TR); Türkyilmaz, Ali, 34460 Istanbul (TR); Mutlu, Onur, 34460 Istanbul (TR)
(74) Representative: Sevinç, Erkan

(56) References cited:
- WO-A1-2010/114504
- WO-A1-2010/114506
- US-A1- 2004 050 385
- US-A1- 2007 215 149
- US-A1- 2009 283 095

## Description

### Field of Invention

The present invention relates to a device for administering dry powder inhalation drugs. The present invention particularly relates to improvements made in the blister advancement mechanism of dry powder inhaler devices.

### Prior Art

Diseases such as asthma, bronchitis, and COLD (Chronic Obstructive Lung Disease) substantially decrease the quality of human life, despite the developments which have been carried out in the diagnosis and therapy thereof in the recent years. It has been proposed to administer medicaments via inhalers for optimizing the treatment of such diseases. The inhaler route of treatment is the most preferred one and it is expected to remain so, as the first option, in the future. The most important advantage of using medicaments via inhalation is based on providing a more efficient therapy by making use of lesser medicaments, delivering higher concentrations of medicaments to the airways, and particularly decreasing the systemic side effects of medicaments. The most important causes of the lack of a satisfactory control of patients albeit the presence of quite efficient treatments against respiratory tract diseases are stated to be as the noncompliance, arising from the inefficient use of inhalers and from inadequate compliance to the physician-recommended treatments.

There have been developed various inhaler devices for administering inhalation drugs nowadays. These devices are basically classified into two groups, i.e. metered dose inhalers and dry powder inhalers. Such type of devices are structurally provided with such basic components as an actuator, counter, housing, lid, lock, etc.. Additionally, powder inhalation drugs are kept in reservoirs or containers such as blisters, capsules, etc.. Blisters are structured from two basic parts, a main layer provided with cavities holding the drug, and a strippable protective layer.

Such devices comprise an outer body and a lid provided thereon, an inner body disposed in the outer body, a blister strip placed in the inner body, a lower holder or reservoir receiving the strip in a rolled form, as well as gears and a gear set connected to each other in a functionally compatible manner to actuate the blister strip so that the main layer thereof comprising the cavities is wound and stored after it is separated into its layers. Push member or trigger mechanism have been developed to actuate this mechanism.

In inhaler devices with a plurality of blisters, a force is exerted to the mechanism actuating the blister by means of the trigger or the push member such that the mechanism is actuated. In practice, however, various problems are encountered in the mechanisms. Some of these are encountered in the triggers actuating the mechanism. These triggers are disadvantageous in terms of use difficulty, and require an extra movable volume on the exterior of the inhaler device. The linear motion of trigger mechanisms, which are advantageous in terms of volume and are slid into the interior of the device, is converted into a rotational motion by means of a gear to which it is connected. In the applications WO2010114506, WO2010114505, for instance, the linear motion performed by a trigger is converted into a rotational motion by means of a wheel. This trigger is slid into a slot in the body of the inhaler device to perform axial motion, and the force generated by this motion is transferred to a blister advancement mechanism by means of a gear with which it meshes. However, the gears providing the transmission and conversion of this motion are exposed to overloads. Additionally, losses are encountered in force transmission when (an) extra gear(s) is/are used. The elimination of this loss, in turn, requires the exertion of an extra amount of force. Another drawback is that slackness occurs between the gears and the contact surfaces. This slackness and extra force exertion, in turn, result in a greater problem in that the gears and wheels in the mechanism become worn out or even break.

US2007/0215149 A1 discloses a dry powder inhaler including: (a) a first generally planar spiral travel path in an inhaler body, wherein the first spiral travel path has a plurality of adjacent curvilinear channels forming lanes with upstanding sidewalls, including an inner lane and an outer lane; and (b) a plurality of discrete sealed microcartridges with substantially rigid bodies disposed in the first travel path, each comprising a pre-metered (typically dose) amount of dry powder, the microcartridges being configured to slidably advance along the first travel path toward an inhalation chamber that merges into an inhalation output port.

The use of the currently available inhaler devices necessitates certain training and practice. The development of inhaler devices always occurs in the form of practical systems, providing the patients with improved convenience of use. Selecting the proper device for a respective patient is also an important issue. Many criteria, such as a patient's cognitive and physical efficiency, ease of use, safety and price, etc. are considered while a device is selected.

In result, there is a novelty required in the field of inhaler devices, providing high-accuracy operation, advantages in terms of cost, volume, and use, as well as minimizing the failures and damages which can occur in the device.

### Objects and Brief Description of Invention

The present invention relates to an improved inhaler device for use with dry powder inhaling purposes, eliminating all aforesaid problems and bringing additional advantages to the relevant prior art.

Accordingly, the main object of the present invention is to provide a dry powder inhaler device, which can be operated at a desired accuracy, can peel off a blister for use, and can perform this process faultlessly as compared to similar devices.

Another object of the present invention is to reduce the amount of force to be exerted to the trigger of the device to operate the same.

A further object of the present invention is to reduce the amount of force to be exerted to the trigger of the device by means of a torque-generating mechanism embodiment.

Another object of the present invention is to prevent the wearing or breaking of the components in the mechanism as a result of reducing the amount of force exerted to the trigger and thus to the gears and the gear mechanism.

In order to achieve the objects referred to above and to emerge in the following detailed description, a dry powder inhaler device is developed which comprises a body and a trigger moving on an axial/linear direction in the body, a main drum into which a blister strip having drug-carrying cavities is placed, a gear set which enables to release a drug in the next cavity of the blister strip to be administered with rotating the main drum and around which a protective layer of the blister strip is wound, and additional gears which are in connection with the main drum and around which a main layer of the blister strip is wound.

The invention is defined in the appended claim 1. The present invention comprises a pivot arm in a moveable connection with the trigger, and a transmission arm which is in a moving connection with the pivot arm and is provided with a transmission wheel on one end thereof to transmit a drive exerted to the trigger to the gear set and to convert the linear motion of the trigger into rotational motion.

The present invention comprises a pivot arm which can move only by rotating around a support/pin connected to the body and coupled to the pivot arm at a point between a first end and a second end of the pivot arm, and which is connected to the trigger from its first end so that an linear motion received from the trigger is converted into a rotational motion on its second end, and a transmission arm having a first end with a wheel coupled to a drum in a rotating manner and a second end having a guide opening to which a transmission pin disposed on the second end of the pivot arm is connected, and transmitting a rotational motion received from the pivot arm to the drum in a reversed direction by reversing the direction of this rotational motion to convert the linear motion of the trigger into rotational motion and transfer this motion to the gear set.

Another preferred embodiment according to the present invention comprises a groove providing the connection of the pivot arm and extending vertically to the axial motion direction of the trigger.

Another preferred embodiment according to the present invention comprises a pin providing a moving connection with the trigger on the pivot arm.

Another preferred embodiment according to the present invention comprises a pin at the second end of the pivot arm to provide a moving connection between the pivot arm and the transmission arm.

In a further preferred embodiment according to the present invention, the main drum is coupled to the transmission wheel disposed on the transmission arm.

A further preferred embodiment according to the present invention comprises a pretensioned spring which restores the trigger to its initial position after it is pushed in axially and released by a user.

Structural and characteristic features, and all advantages of the present invention shall be made clear by means of annexed figures described here below and a detailed description written by making references to said figures; therefore, the present invention must be evaluated by taking into consideration these figures and the detailed description as well.

### Brief Description of Figures

Figure 1 is an illustration of a representative embodiment of an inhaler device according to the present invention.
Figure 2 is a representative embodiment of an outer body and an inner body according to the present invention.
Figure 3 is a representative embodiment of the outer body, inner body, and a mechanism according to the present invention.
Figure 4 is a representative embodiment of the outer body and an the mechanism according to the present invention.
Figure 5 is a representative embodiment of the mechanism according to the present invention.
Figure 6 is a representative embodiment of the mechanism according to the present invention.
Figure 7 is a representative embodiment of the mechanism according to the present invention.
Figure 8 is a representative embodiment of the inner body according to the present invention.

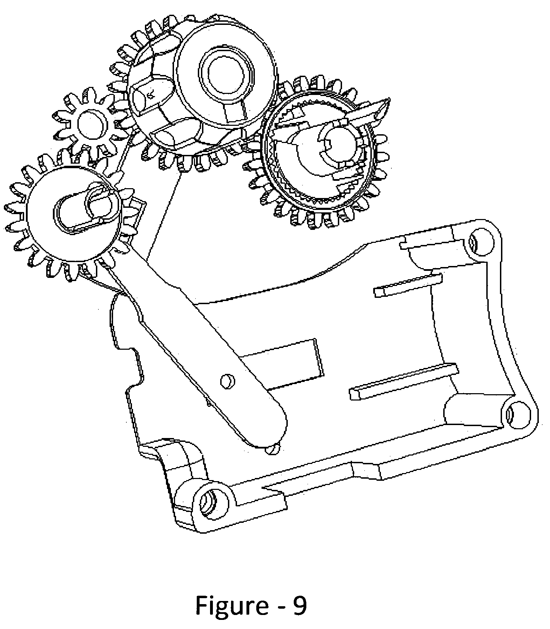

Figure 9 is a representative embodiment of the mechanism according to the present invention.
Figure 10a is a representative embodiment of a main drum according to the present invention.
Figure 10b is a representative embodiment of a transmission wheel, transmission arm, and a pivot arm according to the present invention.
Figure 11 a is a representative embodiment of blister cover winding gears according to the present invention.

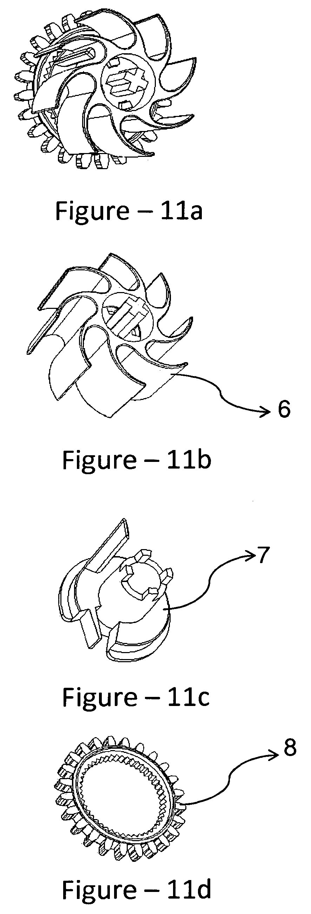

Figure 11 b is a representative embodiment of a blister cover upper winding gear according to the present invention.
Figure 11c is a representative embodiment of a blister cover central winding gear according to the present invention.
Figure 11d is a representative embodiment of a blister cover lower winding gear according to the present invention.

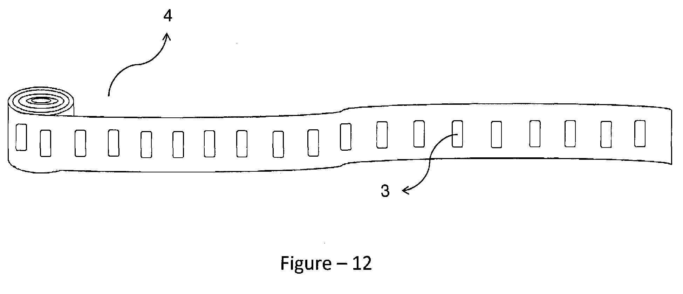

Figure 12 is a representative embodiment of a blister according to the present invention.

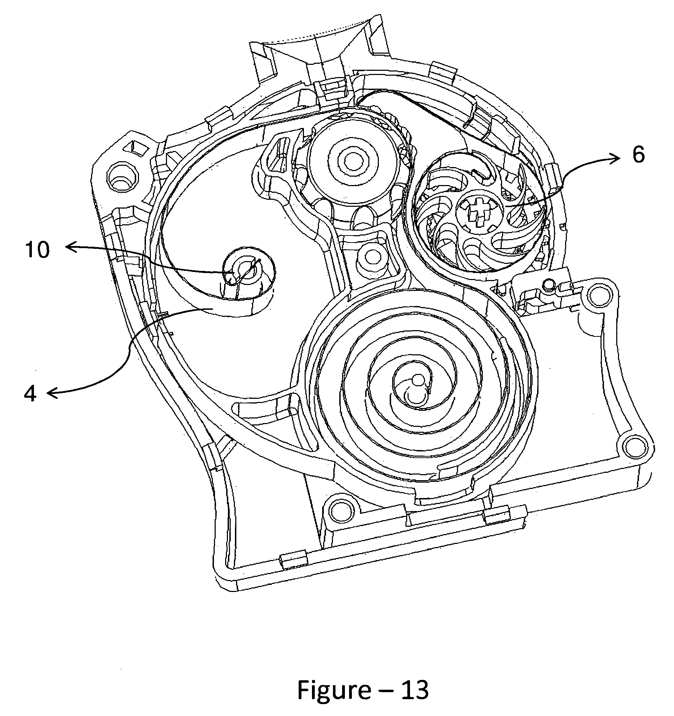

Figure 13 is a representative embodiment of the present invention.

### Reference Numbers in Figures

1. Outer body
2. Trigger
3. Cavity
4. Blister
5. Main drum
6. Blister cover upper winding gear
7. Blister cover central winding gear
8. Blister cover lower winding gear
9. First additional gear
10. Second additional gear
11. Inhaler device
12. Pivot arm
13. Transmission wheel
14. Transmission arm
15. First end of the pivot arm
16. Second end of the pivot arm
17. Support/pin
18. First end of the transmission arm
19. Second end of the transmission arm
20. Transmission pin
21. Guide opening
22. Groove for the pivot arm
23. Pin for the pivot arm
24. Spring
25. Inner body
26. Lower reservoir
27. Left medial reservoir
28. Right medial reservoir
29. Upper reservoir
30. Trigger channel

### Detailed Description of Invention

In the following detailed description, an inhaler device (11) according to the present invention shall be described illustratively by making references to annexed figures, only to make it clear without imposing any restrictions thereon.

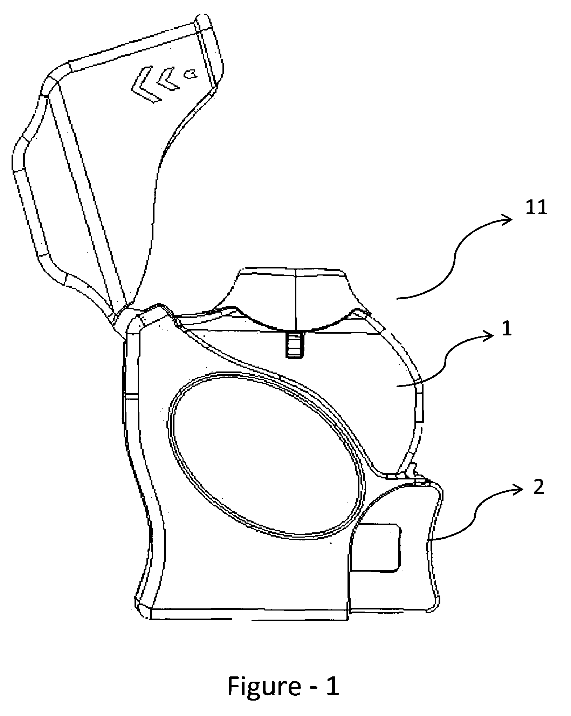

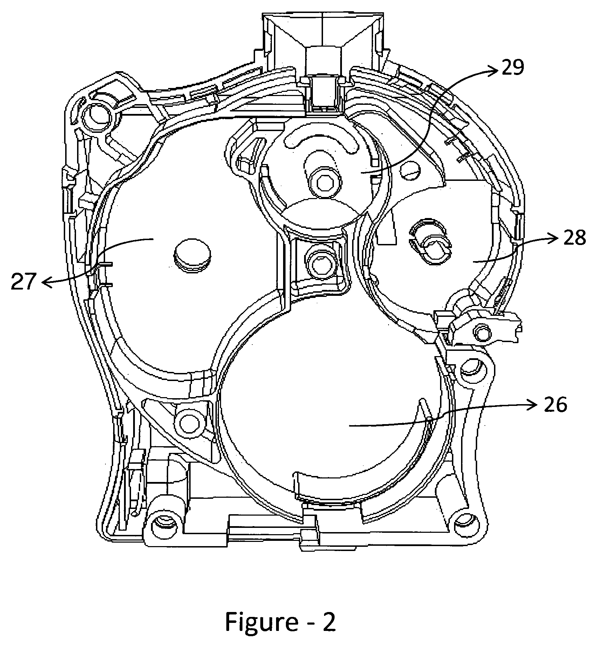

An outer body (1) of the inhaler device (11) according to the present invention as illustrated in figures 1, 2, 4, 10a, 10b, 11 a, 11 b, 11c, and 11 d is obtained by assembling two compatible parts together. The interior of said parts comprises both fastening tabs to fasten them together, and reservoirs and pins, allowing the placement of the mutually connected main drum and transmission wheel (5, 13) and the mutually connected blister cover winding gear set (6, 7, 8) in the outer body (1). The upper part of the body is provided with a mouthpiece having a drug outlet opening. The lower part of the body is provided with a trigger (2) slid into the body.

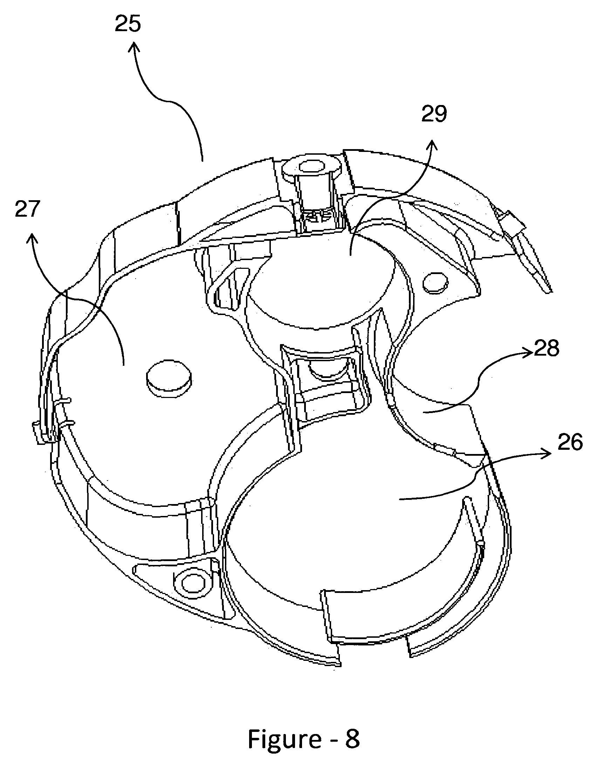

An inner body (25) is positioned in the interior of the outer body (1) to place a blister (4) therein, as illustrated in figures 8 and 13. This single-piece body (25) comprises lower, left and right medial and upper reservoirs (26, 27, 28, 29), respectively, along with recessed surfaces and fastening tabs. An unused blister (4) is stored in the lower reservoir (26), this strip-shaped blister (4) being extended along intermediate channels and subsequently separated into two parts, i.e. a main layer and a protective layer, by means of the mechanism. The main layer with one end fastened to a second additional gear (10) is stored in the left medial reservoir (27) where this second additional gear (10) is positioned. The end of the protective layer, in turn, is fastened to a pin provided on the central winding gear (7).

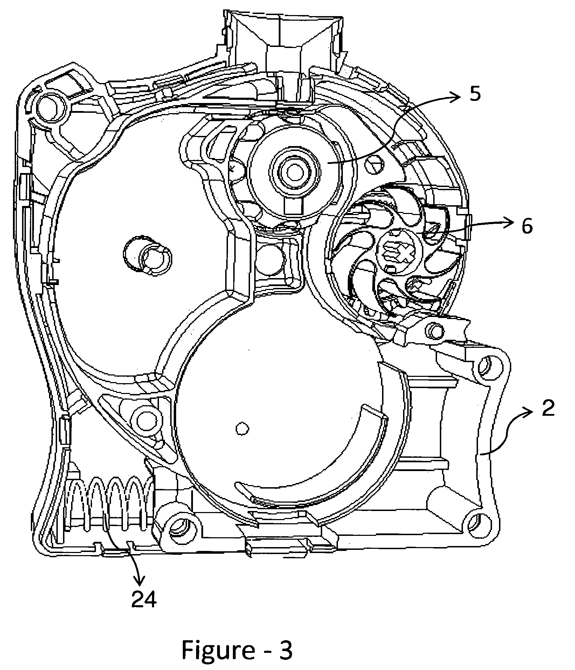

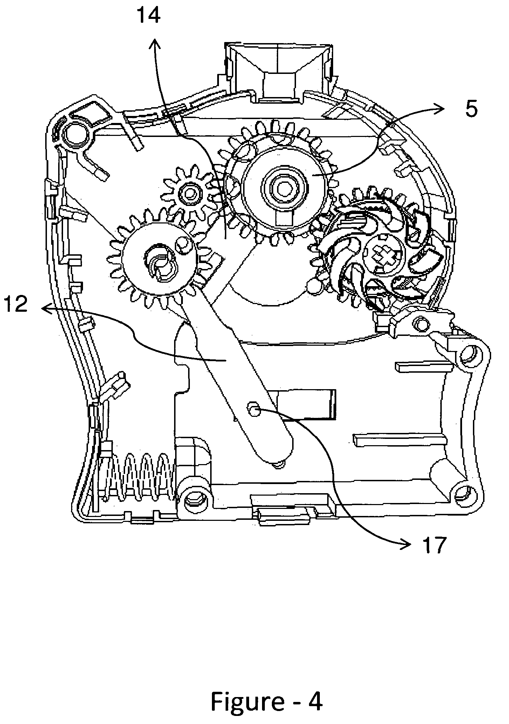

As illustrated in figures 3 and 4, at the beginning of the blister (4) advancement mechanism is provided a trigger (2), slid into a lower side of said outer body (1). The trigger (2) is capable to perform an axial/linear motion in the inner part of the body (1). A grip surface provided on the exterior of the trigger (2), which is the surface by which a user exerts force to push in said trigger, is formed in an incurved manner to provide ease of use. A spring (24) is positioned between the trigger (2) and an inner surface of the outer body (1), this spring becoming compressed (i.e. loaded) when the trigger (2) is pushed into the body (1). The spring (24) has a helical form.

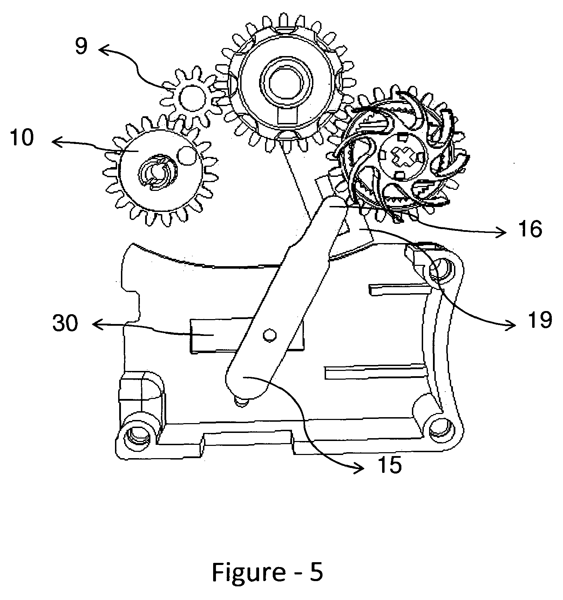

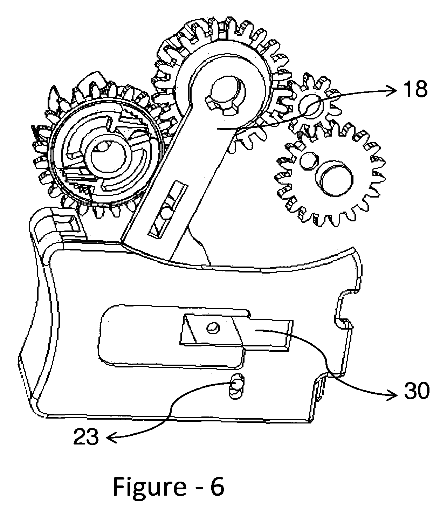

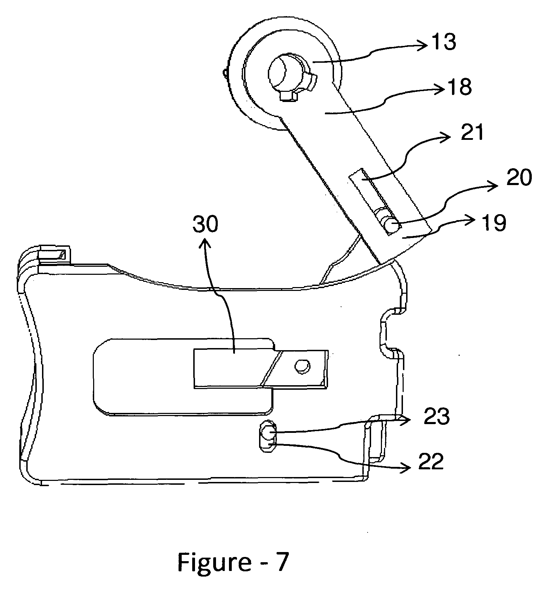

As illustrated in figures 4, 5, 6, and 7, a rectangular cutout (30) is formed on the trigger. A support/pin (17) fixed to the inner part of the body (1) is passed through this cutout. Additionally, a groove (22) which extends perpendicularly to said cutout (30) is provided in the trigger. The central part or a point close to the center of a pivot arm (12) is connected to the pin (17) which passes through the cutout (30) in the body of the trigger (2). This arm (12) is connected to the pin (17) so as to pivot around the pin (17). Additionally, the first end (15) and the second end (16) of the pivot arm are provided with a pin (23, 20) respectively. The pin (23) provided on the first end is connected to the groove (22) extending vertically on the trigger (2). A transmission arm (14) is connected to the pivot arm (12). The pivot arm is capable to move up- and downward in the groove (22). The pivot arm connected as specified above is moved up- or downward so that distance adjustment is made as necessary for the folding and unfolding movement which the pivot arm makes together with the transmission arm (14). Thus the pivot arm and the transmission arm are moved like a scissor to actuate the mechanism. A guide opening (21) provided on the body of this transmission art (14) is connected to the pivot arm by means of a transmission pin (20) provided on the second end of the pivot arm. A wheel (13) is provided on the tip part of the transmission arm. This wheel (13) is engaged to a main drum (5). The main drum (5) is mounted over the wheel (13) so as to move in the same way with the wheel.

According to the details given above, the operation of the device according to the present invention is as follows. Following the opening of the lid of the device, a force is exerted by the user to the grip surface of the trigger. Then the trigger (2) is slid into the interior of the body (1). With the axial/linear motion of the trigger (2), the pivot arm (12) coupled to the trigger is set into motion. The pivot arm is actuated by means of the pin (23), which is provided on the first end of the pivot arm and is engaged to the vertically extending groove (22) on the trigger. The pivot arm (12) rotates around the support/pin (17) which is passed through an opening on the middle part of the pivot arm. This is not a complete rotational motion. The arm performs a rotational motion at an angle lower than 360 degrees. This motion of the pivot arm is transmitted to the transmission arm (14) which is coupled to the pivot arm (12). Under the influence of the pivot arm (12), the transmission arm (14) performs a rotational motion through the wheel (13) with which it is coupled. With this motion of the transmission arm (14), the wheel (13) provided on one end thereof (14) is actuated. With the movement of the wheel (13), the main drum (5) disposed over the wheel is rotated so as to rotate the gears with which it meshes. Thus, a blister placed around the gears is moved and the cavities (3) of the blister (4) are opened.

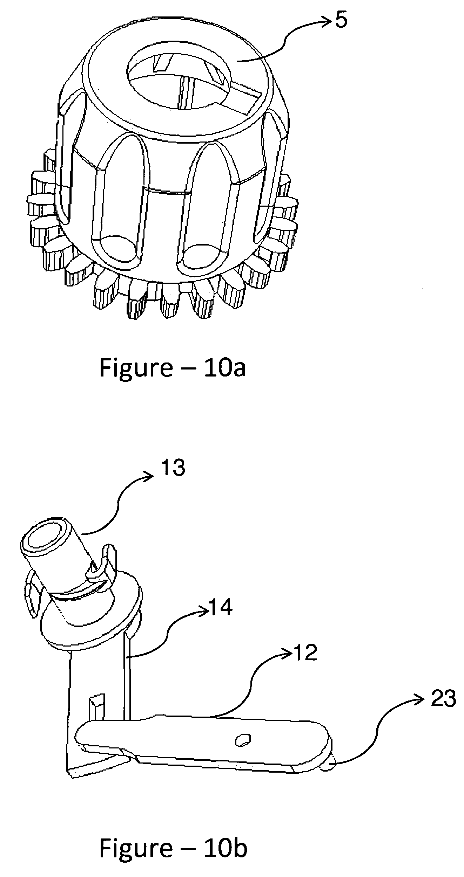

As illustrated in detail in Figure 10, the main drum (5) moves on a single direction and provides only the advancement of the blister (4). The transmission wheel (13) below the main drum is capable to rotate forwards and backwards.

The transmission wheel (13) transmits its rotational motion to the main drum (5) and to the first and second additional gears (9, 10). While the transmission wheel and the main drum (13, 5) rotate counterclockwise, the blister cover lower winding gear (8) and the blister cover central and upper winding gears (7, 6) disposed above the lower winding gear rotate clockwise. The blister (4) in contact with these gears is advanced in the channels of the inner body (25), is passed through and stripped or peeled off between the gear sets so that the drug in the respective cavity is released. The main layer of the blister, now separated into two parts, is rolled in the left medial reservoir (27), whereas the protective or cover layer of the blister is wound around the blister cover upper gear (6).

As a result of sliding the trigger (2) into the interior of the outer body (1), a retaining groove provided on the trigger (2) is coupled and fastened to a locking clips lug provided just over the groove, resulting in the administration of a single dose of medicament. Keeping this slide-in action until the locking position is achieved ensures a complete peeling-off of the blister and an accurate administration of the required dosage amount. As a result of this locking effect, the trigger (2) becomes retained and it remains out-of-use for a short period of time. This slide-in action also causes the spring (24) to become compressed between the trigger (2) and the interior of the outer body (1).

After the user inhales the powder drug, he/she closes the lid of the device, so that a tip part of the lid exerts force to a rear part of the locking clips, the tip thereof is lifted above, and the clips lug and the retaining groove are detached from each other. As a result of this, the spring (24) in a compressed form pushes back the trigger (2) and restores the device for the next use, without requiring any user intervention. While the trigger (2) is displaced backwards, however, the mechanism does not move the blister backwards. It can be seen from the disclosure above, that the device according to the present invention can be operated by a simple single push action of the user. When the lid is closed, the device (11) is set up automatically and is restored for the next use.

The linear motion of the trigger is the sliding motion of the trigger in any direction, including those on the x, y and z axes. This direction is determined according to the design of the device.

The term gear set covers the main drum, transmission wheel, blister cover lower, central, and upper winding gears, and the additional gears, wherein other wheels and gears to be added to the basic elements of the device are also covered under this term.

| **Device** | **F0 (N)** | **FB (N)** | **F1 (N)** | **F15 (N)** | **L (mm)** | **Mb (Nmm** | **fb (N)** | **M1 (Nmm)** | **f1 (N)** | **M15 (Nmm)** | **f15 (N)** |
|---|---|---|---|---|---|---|---|---|---|---|---|
| Device according to the present invention | 11 | 16,7 | 21 | 25 | 20 | 62 | 3,1 | 200 | 10 | 300 | 15 |
| Device according to the prior art | 11 | 21 | 43 | 60 | 6,2 | 62 | 10 | 200 | 32 | 300 | 49 |

- F0:: Trigger force of a device with the mechanism removed
- FB:: Trigger force of an empty device with a complete mechanism, but without a blister
- F1:: First trigger force of a device loaded with a blister
- F15:: Fifteenth trigger force of a device loaded with a blister without the device being subjected to cleaning
- I:: The distance or the arm length which is perpendicular to the rotational axis.
- Mb.....M15:: Force required to rotate the main drum under respective conditions
- fb.......f15: Force exerted to the main actuating lever or gear to rotate the main drum under respective conditions.

As can be seen in the table, the force which is required to be exerted by a user on the trigger of the device according to the present invention to operate the same is reduced almost to half of those of the devices according to the prior art. The torque generated by means of said arms provides a substantial advantage in the push force required to slide in the trigger.

In result, an inhaler device is obtained with the embodiment disclosed above, this device being extremely accurate, reliably-operating, and providing advantages in terms of cost and volume, wherein the force required to operate the device and exerted to the components of the mechanism thereof is reduced. Two arms (12, 14) are used in place of a gear between the trigger (2) and the main drum (5) to give these advantages. The torque generated by means of said arms provides a substantial advantage in the push force required to slide in the trigger. Additionally, since the load exposed by the gears is substantially reduced, the breaking and wearing down of the device components are prevented to a substantial extent. A device is developed by virtue of this embodiment, which can be used even by the elders and children without any difficulty.

The design of components used may be varied in alternative embodiments according to the type of device being produced. In result, the protection scope of the present invention is set forth in appended claims and cannot be restricted to the illustrative disclosures given above, under the detailed description. It is obvious that a person skilled in the relevant art can produce similar embodiments under the light of the foregoing disclosures, without departing from the main principles of the present invention.

## Claims

1. A dry powder inhaler device (11) comprising a body (1) and a trigger (2) moving on an linear direction in the body, a main drum (5) into which a blister (4) strip having drug-carrying cavities (3) is placed, a gear set (6, 7, 8) which enables to release a drug in a subsequent cavity of the blister strip to be administered with rotating the main drum and around which a protective layer or cover of the blister strip is wound, and additional gears (9, 10) which are in connection with the main drum (5) and around which a main layer of the blister (4) strip is wound, **characterized in that** said device comprises
- a pivot arm (12) having a moveable connection with the trigger (2), and
- a transmission arm (14) having a moving connection with the pivot arm, one end of the transmission arm (14) being provided with a transmission wheel (13),
- the pivot arm (12) is rotatable around a support/pin (17) connected to the body (1) and is coupled to said body (1) at a point between a first end (15) and a second end (16) of the pivot arm (12), and said pivot arm (12) is connected to the trigger (2) from its first end (15) such that linear motion received from the trigger (2) is converted into a rotational motion on its second end (16), and
- the transmission arm (14) has a first end (18) with a wheel (13) coupled to a drum (5) in a rotating manner and a second end (19) with a guide opening (21) to which a transmission pin (20) disposed on the second end of the pivot arm (12) is connected, such that said transmission arm (14) transmits rotational motion received from the pivot arm (12) to the drum (5) by reversing the direction of this rotational motion,
such that linear motion of the trigger (2) converted into rotational motion is transferred to the gear set.

2. The dry powder inhaler device (11) according to Claim 1 **characterized in that** said trigger (2) comprises a groove (22) providing the connection of the pivot arm (12) and extending vertically on the trigger (2).

3. The dry powder inhaler device (11) according to any of the preceding claims, **characterized in that** said pivot arm (12) comprises a pin (23) providing a moving connection with the trigger (2).

4. The dry powder inhaler device (11) according to any of the preceding claims, **characterized in that** the second end of the pivot arm (12) comprises a transmission pin (20) to provide a moving connection between the pivot arm (12) and the transmission arm (14).

5. The dry powder inhaler device (11) according to any of the preceding claims, **characterized in that** the main drum (5) is coupled to the transmission wheel (13) disposed on the transmission arm (14).

6. The dry powder inhaler device (11) according to any of the preceding claims, **characterized by** comprising a pre-compressed spring (24) which restores the trigger (2) to its initial position after it is pushed in and released by a user.

7. The dry powder inhaler device (11) according to any of the preceding claims, wherein the pivot arm (12) and the transmission arm (14) are positioned between the main drum (5) and the trigger (2).

## Patentansprüche

1. Trockenpulverinhalationsgerät (11), bestehend aus einem Körper (11) und einem in linearer Bewegungsrichtung im Körper verlaufenden Auslöser (2), einer Haupttrommel (5), in der ein Blisterstreifen (4) mit mit Arzneimittel befüllten Hohlräumen (3) eingesetzt ist, einem Zahnradsatz (6, 7, 8) der die Freisetzung eines zu verabreichenden Arzneimittels aus dem jeweils nächsten Hohlraum des Blisterstreifens bei Drehung der Haupttrommel ermöglicht und um den eine Schutzschicht oder eine Abdeckung des Blisterstreifens herumgewickelt ist, und zusätzlichen Zahnrädern (9, 10), die mit der Haupttrommel (5) in Verbindung stehen und um die eine Hauptschicht des Blisterstreifens (4) herumgeführt ist, **dadurch gekennzeichnet, dass** das Inhalationsgerät folgendes umfasst:
- einen Schwenkarm (12) mit beweglicher Verbindung zum Auslöser (2), und
- einen mit dem Schwenkarm (12) beweglich verbundenen Übertragungsarm (14), wobei an einem Ende des Übertragungsarms (14) eine Übertragungswalze (13) vorgesehen ist,
- der Schwenkarm (12) um eine Halterung/einen Stift (17) herum drehbar ist, welche(r) mit dem Körper (1) verbunden ist und an einer Stelle zwischen einem ersten Ende (15) und einem zweiten Ende (16) des Schwenkarms (12) mit dem Körper (1) gekoppelt ist, und der Schwenkarm (12) über sein erstes Ende (15) mit dem Auslöser (2) verbunden ist, so dass eine vom Auslöser (2) übertragene lineare Bewegung an seinem zweiten Ende (16) in eine Drehbewegung umgewandelt wird, und
- der Übertragungsarm (14) an seinem ersten Ende (18) eine Walze (13) aufweist, welche drehbar mit einer Trommel (5) verbunden ist, und an seinem zweiten Ende (19) mit einer Führungsöffnung (21) versehen ist, in der ein am zweiten Ende (16) des Schwenkarms (12) vorgesehener Übertragungsstift (20) verläuft, so dass der Übertragungsarm (14) eine vom Schwenkarm (12) an ihn übertragene Drehbewegung durch Richtungsumkehr dieser Drehbewegung an die Trommel (5) überträgt, um dadurch die in eine Drehbewegung umgewandelte lineare Bewegung des Auslösers (2) an den Zahnradsatz zu übertragen.

2. Trockenpulverinhalationsgerät (11) nach Anspruch 1, **dadurch gekennzeichnet, dass** der Auslöser (2) mit einer vertikal am Auslöser (2) verlaufenden Nut (22) zur Verbindung des Schwenkarms (12) versehen ist.

3. Trockenpulverinhalationsgerät (11) nach einem der vorhergehenden Ansprüche, **dadurch gekennzeichnet, dass** der Schwenkarm (12) einen Stift (23) umfasst, der eine bewegliche Verbindung zum Auslöser (2) herstellt.

4. Trockenpulverinhalationsgerät (11) nach einem der vorhergehenden Ansprüche, **dadurch gekennzeichnet, dass** das zweite Ende des Schwenkarms (12) einen Übertragungsstift (20) umfasst, der eine bewegliche Verbindung zwischen dem Schwenkarm (12) und dem Übertragungsarm (14) herstellt.

5. Trockenpulverinhalationsgerät (11) nach einem der vorhergehenden Ansprüche, **dadurch gekennzeichnet, dass** die Haupttrommel (5) mit der am Übertragungsarm (14) vorgesehenen Übertragungswalze (13) gekoppelt ist.

6. Trockenpulverinhalationsgerät (11) nach einem der vorhergehenden Ansprüche, **gekennzeichnet durch** eine vorgespannte Feder (24), die den Auslöser (2) nach dessen Betätigung (Eindrücken und Loslassen) **durch** einen Benutzer wieder in seine Ausgangsposition zurückführt.

7. Trockenpulverinhalationsgerät (11) nach einem der vorhergehenden Ansprüche, **dadurch gekennzeichnet, dass** der Schwenkarm (12) und der Übertragungsarm (14) zwischen der Haupttrommel (5) und dem Auslöser (2) angeordnet sind.

## Revendications

1. Dispositif inhalateur à poudre sèche (11) comprenant un corps (1) et une gâchette (2) en mouvement sur une direction linéaire dans le corps, un tambour principal (5) dans lequel une bande alvéolaire (4) ayant des cavités de transport de médicament (3) est placée, un train d'engrenages (6, 7, 8) qui permet de libérer un médicament dans une cavité subséquente de la bande alvéolaire devant être administré en faisant tourner le tambour principal et autour duquel une couche protectrice ou un revêtement protecteur de la bande alvéolaire est enroulé, et des engrenages supplémentaires (9, 10) qui sont en liaison avec le tambour principal (5) et autour desquels une couche principale de la bande alvéolaire (4) est enroulée, **caractérisé en ce que** ledit dispositif comprend :
- un bras pivotant (12) ayant une liaison mobile avec la gâchette (2), et
- un bras de transmission (14) ayant une liaison en mouvement avec le bras pivotant, une extrémité de du bras de transmission (14) étant munie d'une roue de transmission (13),
- le bras pivotant (12) peut tourner autour d'un support/axe (17) relié au corps (1) et est couplé audit corps (1) à un point entre une première extrémité (15) et une seconde extrémité (16) du bras pivotant (12), et ledit bras pivotant (12) est relié à la gâchette (2) à partir de sa première extrémité (15) de telle sorte qu'un mouvement linéaire reçu de la gâchette (2) est converti en un mouvement de rotation sur sa seconde extrémité (16), et
- le bras de transmission (14) a une première extrémité (18) avec une roue (13) couplée à un tambour (5) de manière rotative et une seconde extrémité (19) avec une ouverture de guidage (21) à laquelle un axe de transmission (20) disposé sur la seconde extrémité du bras pivotant (12) est reliée, de telle sorte que ledit bras de transmission (14) transmet un mouvement de rotation reçu du bras pivotant (12) au tambour (5) en inversant le sens de ce mouvement de rotation,
de telle sorte que le mouvement linéaire de la gâchette (2) est converti en mouvement de rotation et transmis au train d'engrenages.

2. Dispositif inhalateur à poudre sèche (11) selon la revendication 1, **caractérisé en ce que** ladite gâchette (2) comprend une rainure (22) assurant la liaison du bras pivotant (12) et s'étendant verticalement sur la gâchette (2).

3. Dispositif inhalateur à poudre sèche (11) selon l'une quelconque des revendications précédentes, **caractérisé en ce que** ledit bras pivotant (12) comprend une goupille (23) assurant une liaison en mouvement avec la gâchette (2).

4. Dispositif inhalateur à poudre sèche (11) selon l'une quelconque des revendications précédentes, **caractérisé en ce que** la seconde extrémité du bras pivotant (12) comprend un axe de transmission (20) pour assurer une liaison en mouvement entre le bras pivotant (12) et le bras de transmission (14).

5. Dispositif inhalateur à poudre sèche (11) selon l'une quelconque des revendications précédentes, **caractérisé en ce que** le tambour principal (5) est couplé à la roue de transmission (13) disposée sur le bras de transmission (14).

6. Dispositif inhalateur à poudre sèche (11) selon l'une quelconque des revendications précédentes, **caractérisé en ce qu'**il comprend un ressort pré-comprimé (24) qui ramène la gâchette (2) à sa position initiale après qu'il a été poussé et libéré par un utilisateur.

7. Dispositif inhalateur à poudre sèche (11) selon l'une quelconque des revendications précédentes, dans lequel le bras pivotant (12) et le bras de transmission (14) sont positionnés entre le tambour principal (5) et la gâchette (2).
